Europäisches Patentamt

European Patent Office

Office européen des brevets

(11) Publication number: **0 159 089**
**A1**

# (12) EUROPEAN PATENT APPLICATION

(21) Application number: **85200544.6**

(22) Date of filing: **04.04.85**

(51) Int. Cl.⁴: **A 61 K 9/22**
**A 61 L 17/00**

(30) Priority: **04.04.84 NL 8401061**

(43) Date of publication of application:
**23.10.85 Bulletin 85/43**

(84) Designated Contracting States:
**AT BE CH DE FR GB IT LI LU NL SE**

(71) Applicant: **Stichting Biomaterials Science Center, VU, "BSC-VU"**
**De Boelelaan 1115**
**NL-1081 HV Amsterdam(NL)**

(72) Inventor: **De Groot, Klaas**
**L. van Wijkplein 6**
**NL-2101 EL Heemstede(NL)**

(74) Representative: **Urbanus, Henricus Maria, Ir. et al,**
**c/o Vereenigde Octrooibureaux Nieuwe Parklaan 107**
**NL-2587 BP 's-Gravenhage(NL)**

(54) A process for preparing an implant material which releases medicines in the body.

(57) Implant material for the release of medicine in bone tissues or soft tissues, comprising a medicine and a carrier therefor, composed of calcium sulphate and calcium mono-hydrogen phosphate.

Croydon Printing Company Ltd.

EP 0 159 089 A1

-1-

A process for preparing an implant material which releases medicines in the body.

This invention relates to a system which after implantation releases a medicine to its surroundings, and which during the process of release is dissolved in full.

It can be inferred from an article by Wahling et al. (The release of gentamycin from polymethylmethacrylate beads, J. Bone Joint Surg. Br. Vol., 1978, 60, 270) that a two-component system consisting of beads of polymethylmethacrylate ("bone cement") in which the antibiotic gentamycin is dissolved is capable of successfully treating osteomyelitis. One disadvantage is that after the release of the antibiotic the beads of bone cement must be surgically removed from the bone. In the literature, therefore, certain methods are indicated of using, instead of the non-resorbing bone cement, a material that is dissolved in the body itself, so that surgical removal is unnecessary.

A first method is the use of beta-whitlockite instead of bone cement. Beta-whitlockite (chemical formula $Ca_3(PO_4)_2$) is, under certain conditions, more or less soluble. The biological dissolving process depends on the way in which the subject product has been sintered. (Sintering is a process - see a Chapter by K. de Groot in the book "Biocompatability of Clinical Implant Materials", Editor D.F. Williams, C.R.C. Press, Boca Raton, USA, 1981, 199 - 224 - the principle of which is that, by heating, powder particles are cemented together via solid-state diffusion, so that from a powder, a solid body is formed). The rate of dissolution is promoted by ensuring that the poweder particles are only connected together by means of small "necks". Eittenmuller et al. (see his paper entitled: Resorbierbare Antibiotik/Antiseptische TCP-keramik zur lokalen Behandlung der Osteomyelitis, L. Koelner Workshop über Hydroxylkeramik, Cologne, December 7, 1983) have used this beta-whitlockite (also referred to as beta-TCP or β-TCP) in a highly porous form as a carrier for an antibiotic, instead of bone cement.

A second method is the use of calcium sulphate (Plaster of Paris). Varlet et al., in the article "Billes de platre de Paris aux antibiotique dans le traitement de l'infection osseuse", in "Revue de Chirurgie Orthopedique", 1983, 69, 239 - 244, have indicated and shown that gypsum, too, can function as a biologically soluble bead. Their method is predicated on gypsum being made up with the medicine to form a solid final product (by adding water), which like Eittenmuller's TCP has a highly porous composition. Other methods are not based on ceramic starting materials, and accordingly fall outside the scope of the present patent application.

Characteristic of both methods is that the medicine ("antibiotic") is released by diffusion, whereafter the remaining implant (the remaining component) is resorbed, so that the surgical removal of the beads is unnecessary. (It follows that the release of the medicine is not determined by the rate of biodegradation of the implant system).

Both methods, however, have certain disadvantages. Beta-TCP has the disadvantage that the dissolving process may have adverse side-effects. The cause is as follows. After being implanted, a porous beta-TCP product, on account of the loose bond between the sintered particles ("weak necks") disintegrates into loose particles, which in turn are carried off by macrophages to neighbouring lymph glands. Depending on the weakness of the bond between the sintered particles, the dissolving process which is thus effected is taking place at a faster or slower rate. (Non-porous beta-whitlockite is hardly resorbed). In certain cases the particles carried off are not intracellularly dissolved before the lymph glands are reached, so that, in that case, the lymph glands are filled with calcium phosphate particles. If no macrophages are present, or not enough, particles are accumulated around the implant, whereby further release is adversely affected. A second disadvantage of beta-TCP is that the dissolving process is biologically determined owing to the essential role of macrophages, so that there is no certainty as to the eventual disappearance of the implant.

Calcium sulphate, too, has a disadvantage: its rate of dissolution is so high that the implant is not always replaced by bone tissue, which may leave a void (Frame, J. Dent., 3, 177, 1975). One advantage, compared with beta-whitlockite, is that the dissolving process is a truly physicochemical, and hence predictable one.

The present invention provides a system which (1) unlike the beta-whitlockite described earlier, is dissolved not via disintegration into small particles, and hence without involving macrophagic removal; and (2) is not dissolved as fast as gypsum, so that bone voids may be left.

The invention is characterized by being a system comprising a matrix of a mixture of $CaSO_4$ and $CaHPO_4$, in variable proportions, produced by heating powders of $CaSO_4$ and $CaHPO_4$ together to the melting point of $CaHPO_4$, i.e., about 700°C, for some minutes. As this mixture has a solubility which, on the one hand, is intermediate that of $CaSO_4$ and that of $CaHPO_4$ (see the above article by K. de Groot) and, on the other hand, is many times higher that that of beta-whitlockite, it is thus achieved that the dissolving process (1) can be varied in rate, and (2) is not brought about via particles to be carried off by macrophages. To the matrix, heated to form a microporous mass, the desired medicine can be added by means of impregnation.

- 4 -

C L A I M S
============

1.   Implant material for the release of medicine in bone tissues or
soft tissues, comprising a medicine and a carrier therefor, characterized
in that the carrier is composed of calcium sulphate and calcium monohydrogen phosphate.

2.   A process for producing an implant material for the release of
medicine, comprising a medicine and a carrier therefor, characterized
by heating a mixture of calcium sulphate and calcium monohydrogen phosphate to form a microporous mass, and incorporating a medicine therein
by impregnation.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 087 662 (MERCK PATENT GmbH)<br>* Page 4, line 7 - page 6, line 17 * | 1-2 | A 61 K 9/22<br>A 61 L 17/00 |
| A | GB-A-2 093 348 (LEO PHARMACEUTICAL PROD. LTD.)<br>* Page 4, lines 24-105 * | 1-2 | |
| A | GB-A- 630 439 (ETHAN ALLAN BROWN)<br>* Page 2, line 108 - page 3, line 34 * | 1-2 | |

TECHNICAL FIELDS SEARCHED (Int. Cl.4)

A 61 K
A 61 L

The present search report has been drawn up for all claims

| Place of search | Date of completion of the search | Examiner |
|---|---|---|
| THE HAGUE | 11-07-1985 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82